# EUROPÄISCHE PATENTSCHRIFT

(11) **EP 0 749 959 B1**
(45) Veröffentlichungstag und Bekanntmachung des Hinweises auf die Patenterteilung: **16.02.2000**
(21) Anmeldenummer: 96109318.4
(22) Anmeldetag: 11.06.1996
(51) Int. Cl.: C07C 269/08, C07C 271/24

(54) **Verfahren zur Entfernung von Nebenprodukten aus Diurethanen**
Process removing by-products from diurethanes
Procédé pour éliminer des sous-produits des diuréthanes

(30) Priorität: 23.06.1995 DE 19523386
(43) Veröffentlichungstag der Anmeldung: 27.12.1996
(73) Patentinhaber: BAYER AG, 51368 Leverkusen (DE)
(72) Erfinder: Deibele, Ludwig, Dr., 50677 Köln (DE); Wilmes, Oswald, Dr., 51061 Köln (DE); Dahmer, Jürgen, Dr., 51061 Köln (DE)

(56) Entgegenhaltungen:
- EP-A- 0 126 299
- EP-A- 0 126 300
- EP-A- 0 568 782

## Beschreibung

Die vorliegende Erfindung betrifft ein neues Verfahren zur Entfernung von niedrigsiedenden Nebenprodukten aus nach phosgenfreien Verfahren hergestellten Diurethanen.

Diurethane auf Basis von einfachen Diisocyanaten und niedrigsiedenden Alkoholen oder Phenol lassen sich thermisch in die ihnen zugrundeliegenden Diisocyanate spalten. Eine bekannte Methode der phosgenfreien Herstellung von organischen Diisocyanaten besteht daher in der phosgenfreien Herstellung der Diurethane und deren anschließende thermische Spaltung. Zur phosgenfreien Herstellung von Diurethanen sind beispielsweise die Umsetzung von Diaminen mit Harnstoff und Alkohol (EP 18 586, EP 27 940, EP 27 953, EP 126 299, EP 126 300, EP 355 443, EP 568 782, EP 566 925), mit Carbamidsäureestern und gegebenenfalls Alkohol (EP 18 588, EP 27 952) sowie mit Kohlensäureestern (EP 323 514) bekannt. Die so hergestellten Diurethane enthalten neben einem Hochsiederanteil (z.B. Oligo- bzw. Polyharnstoffe) auch tiefer als die Diurethane siedende Verunreinigungen (Kohlensäurederivate). Zur Isolierung der Diurethane werden im allgemeinen gängige Trenntechniken angewandt.

Nach EP 18 586 erfolgt die Reinigung nach Abfiltrieren von Feststoffen z.B. durch Abdestillieren des überschüssigen Alkohols und/oder des Lösemittels sowie des als Nebenprodukt gebildeten bzw. im Überschuß eingesetzten Carbamidsäureesters. Über die Reinheit der erhaltenen Produkte wird keine Angabe gemacht. Nachteilig ist hierbei, daß hochsiedende Verunreinigungen im Produkt verbleiben. Zur vollständigen Abtrennung der niedriger als das Diurethan siedenden Nebenkomponenten mittels Destillation muß die Reaktionsmischung über eine gewisse Zeit thermisch belastet werden, wodurch weitere Zersetzungsprodukte gebildet werden können.

Als weitere Reinigungsmöglichkeiten werden Kristallisations- bzw. Fällungsverfahren angeführt, die jedoch wegen des höheren verfahrenstechnischen Aufwands für ein technisches Verfahren weniger wirtschaftlich sind.

Gemäß EP 323 514, EP 27 940, EP 27 952 und EP 27 953 erfolgt die Aufarbeitung insbesondere destillativ, wobei nach Abdestillieren von niedriger siedenden Lösungsmitteln, Hilfsmitteln, Edukten oder Zwischenprodukten die Urethane im allgemeinen als letzte Fraktion oder als Destillationsrückstand anfallen. Vor dieser destillativen Aufarbeitung der Produkte können erforderlichenfalls unlösliche Bestandteile (z.B. unlösliche Katalysatoren) abfiltriert werden.

Nachteilig ist hierbei, daß sich bei der Destillation wegen der thermischen Belastung Zersetzungserscheinungen an den Produkten einstellen können. Dies trifft besonders bei hochsiedenden Diurethanen zu. Ferner können die gewünschten Produkte durch partielle Zersetzung der Nebenbestandteile während der Destillation verunreinigt werden.

Gemäß EP 355 443 und EP 568 782 erfolgt die Aufarbeitung durch destillative Entfernung von nicht umgesetztem Alkohol. Anschließend erfolgt in einem Dünnschichtverdampfer die Abtrennung von N-unsubstituiertem Carbamat und Dialkylcarbonat. Als letzte Destillationsfraktion wird das Diurethan abgenommen.

In EP 126 299 und EP 126 300 wird ein Verfahren beschrieben, bei dem aus einer Reaktionsmischung zunächst ein Lösemittel (Alkohol) abdestilliert wird. In einer zweiten Stufe werden Reste des Lösungsmittels sowie Kohlensäurederivate Dialkylcarbonat und/oder Carbamidsäurealkylester, die aus dem Herstellungsprozeß stammen, in einer Strippkolonne mit Inertgas abgetrennt. Die Temperaturen betragen hierbei bis 200°C, Zersetzungsreaktionen können also nicht ausgeschlossen werden. Für ein technisches Verfahren bedeutet es zudem einen zusätzlichen Aufwand, da vor Abgabe des Inertgases an die Umwelt eine entsprechende Reinigung erforderlich ist. Das so gereinigte Produkt enthält gegebenenfalls Oligoharnstoffpolyurethane, die einem weiteren Destillationsschritt, z.B. in einem Dünnschichtverdampfer, als Sumpf ausgeschleust werden. Hier werden sogar Temperaturen bis 300°C angewendet.

Gemäß EP 566 925 gelingt die Abtrennung von Alkohol, Dialkylcarbonat und Carbamidsäurealkylester beispielhaft durch Entspannung vom Druckniveau der Herstellstufe (12 bar) auf einen Druck von 50 mbar. Abgesehen davon, daß dieses Verfahren technisch sehr aufwendig ist, gelingt, wie eigene Versuche gezeigt haben, die vollständige Abtrennung von Dialkylcarbonat bzw. Carbamidsäurealkylester nicht, das Produkt enthält noch bis zu 5 % dieser Nebenkomponenten, die bei der thermischen Urethanspaltung noch in die gewünschte Isocyanatkomponente gelangen können.

Die der vorliegenden Erfindung zugrundeliegende Aufgabe bestand darin, ein schonendes Verfahren zur Entfernung von niedrigsiedenden Nebenprodukten aus durch phosgenfreie Verfahren hergestellten Diurethanen zur Verfügung zu stellen, welches einerseits eine praktisch vollständige Entfernung dieser Nebenprodukte ermöglicht und andererseits das Risiko einer unerwünschten vorzeitigen thermischen Spaltung der Diurethane ausschließt.

Die Aufgabe konnte mit der Bereitstellung des nachstehend näher beschriebenen erfindungsgemäßen Verfahrens gelöst werden. Das neue Verfahren beruht auf dem Prinzip, die niedrigsiedenden Nebenprodukte durch eine Trägerdampfdestillation mittels Alkohol aus den Diurethanen zu entfernen, wobei einerseits Temperaturen zur Anwendung gelangen, die deutlich unter den üblichen Spalttemperaturen liegen und andererseits die Verwendung von Alkohol zur Herstellung des Trägerdampfs einer unerwünschten Spaltung im Sinne des Massenwirkungsgesetzes entgegenwirkt.

Gegenstand der Erfindung ist ein Verfahren zur Entfernung von niedrigsiedenden Nebenprodukten aus nach phosgenfreien Verfahren hergestellten Diurethanen der allgemeinen Formel (I)

R-O-CO-NH-R'-NH-CO-O-R (I)

in welcher
- R: für einen Alkylrest mit 1 bis 6 Kohlenstoffatomen, einen Cycloalkylrest mit 5 bis 6 Kohlenstoffatomen oder einen Phenylrest steht
und
- R': für den Rest steht, wie er durch Entfernung der Isocyanatgruppen aus einem organischen Diisocyanat des Molekulargewichtsbereichs 140 bis 300 steht,
durch eine Trägerdampfdestillation unter Gewinnung der zu entfernenden niedrigsiedenden Nebenprodukte als Trägerdampfdestillat und der von den Nebenprodukten befreiten Diurethane als Trägerdampfdestillationsrückstand, dadurch gekennzeichnet, daß man als Trägerdampf einen Alkoholdampf verwendet.

Ausgangsmaterialien für das erfindungsgemäße Verfahren sind technische Gemische, wie sie bei der phosgenfreien Herstellung von Diurethanen der obengenannten allgemeinen Formel (I) gemäß den Verfahren der bereits vorstehend genannten Veröffentlichung anfallen. Die Hauptprodukte dieser technischen Gemische entsprechen der obengenannten allgemeinen Formel (I), wobei insbesondere solche bevorzugt sind, für welche
- R: für einen Alkylrest mit 1 bis 4 Kohlenstoffatomen steht und
- R': für einen aliphatischen oder cycloaliphatischen Kohlenwasserstoffrest mit 6 bis 15 Kohlenstoffatomen steht und insbesondere den die Isocyanatgruppen von 1,6-Diisocyanatohexan, von Bis-(4-isocyanatocyclohexyl)-methan, von 1-Isocyanato-3,3,5-trimethyl-5-isocyanatomethyl-cyclohexan oder von 2-Methyl- und/oder 4-Methyl-1,3-diisocyanatocyclohexan (hydriertes Toluylendiisocyanat) verknüpfenden Kohlenwasserstoffrest darstellt.

Für das erfindungsgemäße Verfahren einzusetzende Ausgangsmaterialien sind beispielsweise solche, deren Hauptkomponente der Formel (I) aus Butandiurethan-1,4, 1-Methyl-pentandiurethan-1,5, Hexandiurethan-1,6, 2,2,4- bzw. 2,4,4-Trimethylhexandiurethan-1,6, Cyclohexandiurethan-1,3, Cyclohexandiurethan-1,4, 2-Methyl- und/oder 4-Methylcyclohexandiurethan-1,3 (hydriertes Toluylendiurethan), 1,3- und 1,4-Bis-Alkoxycarbonylaminocyclohexan, Bis-(4-alkoxycarbonylaminocyclohexyl)-methan, Bis-(4-alkoxycarbonylamino-3-methylcyclohexyl)-methan, 3-Alkoxycarbonylaminomethyl-3,5,5-trimethyl-cyclohexylurethan (Isophorondiurethan) oder 3- und/oder 4-Alkoxycarbonylaminomethyl-1-methylcyclohexylurethan besteht, wobei die diesen Diurethanen zugrundeliegende Alkoholkomponente insbesondere aus Methanol, Ethanol oder n-Butanol besteht.

Besonders bevorzugt werden solche technischen Gemische eingesetzt, deren Hauptkomponente der Formel (I) aus 1,6-Bis-(C₁-C₄-alkoxycarbonylamino)-hexan (HDU), 1-(C₁-C₄-Alkoxycarbonylamino)-3,3,5-trimethyl-5-(C₁-C₄-alkoxycarbonylaminomethyl)-cyclohexan (IPDU) oder aus Bis-[4-(C₁-C₄-alkoxycarbonylamino)cyclohexyl]-methan besteht. Die bei den unterschiedlichen phosgenfreien Herstellungsverfahren von Diurethanen der Formel (I) entstehenden technischen Gemische enthalten im allgemeinen, jeweils bezogen auf das Gewicht der Diurethane der Formel (I), bis zu 25, vorzugsweise bis zu 15 Gew.-% an Nebenprodukten wie beispielsweise Carbamidsäurealkylestern und/oder Dialkylcarbonaten, die einen unter dem Siedepunkt der Diurethane der Formel (I) liegenden Siedepunkt aufweisen und bis zu 25, vorzugsweise bis zu 15 Gew.-% an Nebenprodukten wie beispielsweise solchen der Formel

ROCONH-R'-[NHCONH-R']ₙ-NHCOOR (n ≥ 1)

die einen über dem Siedepunkt der Dialkylurethane der Formel (I) liegenden Siedepunkt aufweisen. Im allgemeinen werden die Rohgemische nach ihrer Herstellung destillativ von überschüssigem Alkohol bis auf einen Restgehalt, bezogen auf das Gewicht der Diurethane der Formel (I), von maximal 5 Gew.-% befreit.

Bei den dem erfindungsgemäßen Verfahren zuzuführenden Ausgangsgemischen kann es sich somit um solche, von überschüssigem Alkohol weitgehend befreite Rohgemische handeln. Es ist jedoch auch möglich, beim erfindungsgemäßen Verfahren solche Ausgangsgemische einzusetzen, die vorab von den genannten höhersiedenden Nebenprodukten befreit worden sind. Derartige Gemische können beispielsweise dergestalt erhalten werden, daß man die bei den Herstellungsverfahren anfallenden Gemische zunächst vom überschüssigen Alkohol befreit und anschließend einer Dünnschichtdestillation unterzieht, wobei alle Produkte, die tiefer als die genannten hochsiedenden Produkte sieden, als Dünnschichtdestillat gewonnen werden. Dieses Dünnschichtdestillat enthält dann neben den Diurethanen der Formel (I) die genannten tiefer als diese siedenden Nebenprodukte und dient als Ausgangsmaterial beim erfindungsgemäßen Verfahren. Zur Durchführung der erfindungsgemäßen Alkoholdampfdestillation dienen

Dämpfe von Alkoholen der Formel (II)

R''-OH (II),

in welcher
- R'': für einen Alkylrest mit 1 bis 6 Kohlenstoffatomen oder einen Cycloalkylrest steht.

Vorzugsweise entspricht die Bedeutung von R'' der Bedeutung von R.

Die Durchführung des erfindungsgemäßen Verfahrens erfolgt vorzugsweise in Destillationskolonnen, die mit Böden und/oder mit Packungen gefüllt sind. Das von den niedrigsiedenden Nebenprodukten zu befreiende Ausgangsmaterial wird hierbei als Flüssigkeit oben in die Kolonne eingespeist, während der Alkoholdampf unten in die Kolonne geleitet wird. Der Dampf strömt im Gegenstrom zur Flüssigkeit durch die Kolonne und belädt sich dabei mit den leichter als das Diurethan siedenden Nebenprodukten. Er verläßt die Kolonne über Kopf und wird anschließend kondensiert. Das von den Leichtsiedern befreite Diurethan wird als Flüssigkeit dem Sumpf der Kolonne entnommen.

Der Betriebsdruck der Kolonne muß so gewählt werden, daß der zu diesem Druck gehörende Siedepunkt des Alkohols (II) oberhalb des Festpunkts des Diurethans liegt. Im allgemeinen liegt der Druck unterhalb Normaldruck (1013 mbar). Die Temperatur am Kopf der Kolonne liegt im allgemeinen bei 100 bis 200°C. Die Temperatur des Alkoholdampfs liegt im allgemeinen 10 bis 50°C über dem Siedepunkt des Alkohols beim entsprechenden Druck. Die Menge des zur Herstellung des Trägerdampfs verwendeten Alkohols wird im übrigen so bemessen, daß das Volumenverhältnis, jeweils bezogen auf Flüssigkeit, von Trägerdampf-Alkohol zu Ausgangsmaterial (Niedrigsieder und gegebenenfalls Hochsieder enthaltende Diurethane der Formel (I)) bei 0,3:1 bis 5:1, vorzugsweise 0,5:1 bis 2:1 liegt.

Der Gehalt an Alkohol im gereinigten Diurethan läßt sich durch die Überhitzung des Alkoholdampfes variieren. Er liegt vorzugsweise zwischen 0 und 10 %. Bei 0 % muß die Überhitzung der Alkoholdämpfe so groß sein, daß genügend Energie zur Verdampfung der Leichtsieder bereit stehen.

Enthält das zur Trägerdampfdestillation eingesetzte Ausgangsmaterial noch Nebenkomponenten, deren Siedepunkt oberhalb dem des zu reinigenden Diurethans liegt, so kann im Anschluß an die Reinigung entsprechend dem erfindungsgemäßen Verfahren noch eine Hochsiederabtrennung nach an sich bekannten Verfahren, beispielsweise durch Destillation, vorzugsweise durch Dünnschichtdestillation unter Isolierung der Hochsieder als Destillationsrückstand erfolgen.

In dem nachstehenden Beispiel beziehen sich alle Prozentangaben auf das Gewicht.

### Beispiel

Der Betrieb der Trägerdampfdestillation (Füllkörperkolonne mit Durchmesser 50 mm und Füllhöhe 2 m) erfolgt bei 150 mbar. Auf den Kolonnenkopf wurden 500 ml/h Ausgangsmaterial bestehend aus 1,6-Bis-(n-butoxycarbonylamino)-hexan mit einem Gehalt, bezogen auf dieses Diurethan, an leichter siedenden Nebenprodukten von 3,5 Gew.-% (2,2 Gew.-% n-Butylcarbamat und 1,3 Gew.-% Di-n-butylcarbonat) aufgegeben. Als Trägerdampf wurden 500 ml/h (bezogen auf Flüssigkeit) auf 150°C überhitztes n-Butanol verwendet und unten der Kolonne zugeführt. Das entspricht einem Volumenverhältnis von Zulauf zu Trägerdampf von 1:1. Am oberen Ende der Trägerdampfdestillation war der n-Butanol-Dampf mit 3,5 % an leichter Siedenden (2,2 % Butylcarbamat, 1,3 % Dibutylcarbonat) beladen. Das gereinigte Hexandiurethan-1,6 floß dagegen am Kolonnensumpf in reiner Form ab, die genannten leichter siedenden Nebenkomponenten waren nicht mehr nachweisbar (Nachweisgrenze jeweils 0,1 %).

## Patentansprüche

1. Verfahren zur Entfernung von niedrigsiedenden Nebenprodukten aus nach phosgenfreien Verfahren hergestellten Diurethanen der allgemeinen Formel (I)
R-O-CO-NH-R'-NH-CO-O-R (I)
in welcher
R für einen Alkylrest mit 1 bis 6 Kohlenstoffatomen, einen Cycloalkylrest mit 5 bis 6 Kohlenstoffatomen oder einen Phenylrest steht
und
R' für den Rest steht, wie er durch Entfernung der Isocyanatgruppen aus einem organischen Diisocyanat des Molekulargewichtsbereichs 140 bis 300 steht,
durch eine Trägerdampfdestillation unter Gewinnung der zu entfernenden niedrigsiedenden Nebenprodukte als Trägerdampfdestillat und der von den Nebenprodukten befreiten Diurethane als Trägerdampfdestillationsrückstand, dadurch gekennzeichnet, daß man als Trägerdampf einen Alkoholdampf verwendet.

2. Verfahren gemäß Anspruch 1, dadurch gekennzeichnet, daß man als Trägerdampf den Dampf des in dem Diurethan chemisch gebundenen Alkohols der Formel (II)
R-OH (II)
verwendet.

3. Verfahren gemäß Anspruch 1 und 2, dadurch gekennzeichnet, daß die Diurethane der Formel (I) entsprechen, wobei
R für einen C₁-C₄-Alkylrest steht und
R' für den die die Isocyanatgruppen von 1,6-Diisocyanatohexan, von Bis-(4-isocyanatocyclohexyl)-methan, von 1-Isocyanato-3,3,5-trimethyl-5-isocyanatomethyl-cyclohexan oder von 2-Methyl- und/oder 4-Methyl-1,3-diisocyanatocyclohexan verknüpfenden Kohlenwasserstoffreste steht.

4. Verfahren gemäß Anspruch 1 bis 3, dadurch gekennzeichnet, daß man die Trägerdampfdestillation unter Einspeisung des Ausgangsmaterials und Entnahme des mit Nebenprodukten beladenen Trägerdampfes am Kopf und Einspeisung des Trägerdampfs und Entnahme des Verfahrensprodukts am Sumpf der Kolonne durchführt.

5. Verfahren gemäß Anspruch 1 bis 4, dadurch gekennzeichnet, daß man als Ausgangsmaterial das Rohprodukt einer phosgenfreien Herstellung von Diurethanen der Formel (I) handelt, welches, jeweils bezogen auf Diurethan der Formel (I),
(i) bis zu 5 Gew.-% an Alkohol der Formel (II),
(ii) bis zu 25 Gew.-% an Nebenprodukten mit einem unter dem Siedepunkt des Diurethans der Formel (I) liegenden Siedepunkt und
(iii) bis zu 25 Gew.-% an Nebenprodukten mit einem über dem Siedepunkt des Diurethans der Formel (I) liegenden Siedepunkt
aufweist.

6. Verfahren gemäß Anspruch 5, dadurch gekennzeichnet, daß das Ausgangsmaterial vorab durch Destillation von den Nebenprodukten mit einem über dem Siedepunkt des Diurethans der Formel (I) liegenden Siedepunkt befreit worden ist.

## Claims

1. A process for removing low-boiling by-products from diurethanes, prepared by a phosgene-free method, of the general formula (I)
R-O-CO-NH-R'-NH-CO-O-R (I)
in which
R represents an alkyl group with 1 to 6 carbon atoms, a cycloalkyl group with 5 to 6 carbon atoms or a phenyl group
and
R' represents a group which may be produced by removing the isocyanate groups from an organic diisocyanate with a molecular weight in the range 140 to 300,
by means of carrier distillation with recovery of the low-boiling by-products being removed as carrier distillate and the diurethanes freed of by-products as the carrier distillation residue, characterised in that an alcohol vapour is used as carrier.

2. A process according to claim 1, characterised in that vapour of the alcohol of the formula (II)
R-OH (II)
which is chemically bonded in the diurethane is used as the carrier.

3. A process according to claims 1 and 2, characterised in that the diurethanes correspond to the formula (I), wherein
R represents a C₁-C₄-alkyl group and
R' represents the hydrocarbon group linking the isocyanate groups in 1,6-diisocyanatohexane, bis-(4-isocyanatocyclohexyl)-methane, 1-isocyanato-3,3,5-trimethyl-5-isocyanatomethyl-cyclohexane or 2-methyl- and/or 4-methyl-1,3-diisocyanatocyclohexane.

4. A process according to claims 1 to 3, characterised in that carrier distillation is performed by feeding the starting material to and removing the carrier which contains by-products from the head and feeding the carrier to and removing the process product from the bottom of the column.

5. A process according to claims 1 to 4, characterised in that the starting material used is the crude product from a phosgene-free production of diurethanes of the formula (I), which has, each with respect to the diurethane of the formula (I),
(i) up to 5 wt.% of alcohol of the formula (II),
(ii) up to 25 wt.% of by-products with a boiling point below that of the diurethane of the formula (I) and
(iii) up to 25 wt.% of by-products with a boiling point above that of the diurethane of the formula (I).

6. A process according to claim 5, characterised in that by-products with a boiling point above that of the diurethane with the formula (I) have previously been removed from the starting material by distillation.

## Revendications

1. Procédé pour éliminer les produits d'accompagnement à bas point d'ébullition de diuréthanes préparés sans phosgène et répondant à la formule générale (I)
R-O-CO-NH-R'-NH-CO-O-R (I)
dans laquelle
R représente un groupe alkyle en C₁-C₆, cycloalkyle en C₅-C₆ ou phényle,
et
R' représente le radical obtenu par élimination des groupes iscocyanates d'un diisocyanate organique de poids moléculaire 140 à 300,
par une distillation à la vapeur d'entraînement avec isolement des produits d'accompagnement volatils à éliminer en distillat à la vapeur et les diuréthanes débarrassés des produits d'accompagnement en résidu de la distillation à la vapeur, caractérisé en ce que la vapeur d'entraînement est la vapeur d'un alcool.

2. Procédé selon la revendication 1, caractérisé en ce que l'on utilise en tant que vapeur d'entraînement la vapeur de l'alcool combiné chimiquement dans le diuréthane, répondant à la formule (II)
R-OH (II)

3. Procédé selon les revendications 1 et 2, caractérisé en ce que les diuréthanes répondent à la formule (I) dans laquelle
R représente un groupe alkyle en C1-C4 et
R' représente les radicaux hydrocarbonés reliant les groupes isocyanates du 1,6-diisocyanatohexane, du bis(4-isocyanatocyclohexyl)méthane, du 1-isocyanato-3,3,5-triméthyl-5-isocyanatométhyl-cyclohexane ou du 2-méthyl- et/ou 4-méthyl-1,3-diisocyanatocyclohexane.

4. Procédé selon les revendications 1 à 3, caractérisé en ce que l'on procède à la distillation à la vapeur d'entraînement en introduisant la matière première et en prélevant la vapeur d'entraînement chargée des produits d'accompagnement en tête et en introduisant la vapeur d'entraînement et en évacuant le produit purifié en pied de colonne.

5. Procédé selon les revendications 1 à 4, caractérisé en ce que le produit de départ est le produit brut d'une préparation sans phosgène de diuréthanes de formule (I) qui, par rapport à ce dernier, contient
(i) jusqu'à 5 % en poids de l'alcool de formule (II)
(ii) jusqu'à 25 % en poids de produits d'accompagnement ayant un point d'ébullition inférieur à celui du diuréthane de formule (I) et
(iii) jusqu'à 25 % en poids de produits d'accompagnement ayant un point d'ébullition supérieur à celui du diuréthane de formule (I)

6. Procédé selon la revendication 5, caractérisé en ce que le produit de départ est débarrassé au préalable par distillation des produits d'accompagnement ayant un point d'ébullition supérieur à celui du diuréthane de formule (I)
